# EUROPEAN PATENT SPECIFICATION

(11) **EP 3 562 808 B1**
(45) Date of publication and mention of the grant of the patent: **11.05.2022**
(21) Application number: 17887857.5
(22) Date of filing: 22.12.2017
(51) Int. Cl.: C07D 231/14, C07D 231/38, A01N 43/56, C07D 401/04

(54) **PROCESSES FOR THE PREPARATION OF PESTICIDAL COMPOUNDS**
VERFAHREN ZUR HERSTELLUNG VON PESTIZIDVERBINDUNGEN
PROCÉDÉS DE PRÉPARATION DE COMPOSÉS PESTICIDES

(30) Priority: 29.12.2016 US 201662440178 P
(43) Date of publication of application: 06.11.2019
(73) Proprietor: Corteva Agriscience LLC, Indianapolis, IN 46268 (US)
(72) Inventor: YANG, Qiang, Indianapolis, Indiana 46268 (US); ZHANG, Yu, Indianapolis, Indiana 46268 (US); LORSBACH, Beth, Indianapolis, Indiana 46268 (US); LI, Xiaoyong, Midland, Michigan 48674 (US); ROTH, Gary, Midland, Michigan 48674 (US)
(74) Representative: f & e patent
(86) International application number: PCT/US2017/068250
(87) International publication number: WO 2018/125816

(56) References cited:
- WO-A1-2015/058028
- US-A1- 2012 095 023
- US-A1- 2013 109 566
- US-A1- 2015 112 074
- US-A1- 2015 112 077
- US-A1- 2015 336 929
- US-A1- 2016 152 594
- US-A1- 2016 152 594
- US-A1- 2016 318 924
- US-A1- 2016 332 987

## Description

### CROSS-REFERENCE TO RELATED APPLICATIONS

This application claims priority under 35 U.S.C. § 119(e) to U.S. Provisional Application Serial No. 62/440,178 filed December 29, 2016.

### TECHNICAL FIELD OF THE DISCLOSURE

This application relates to efficient and economical synthetic chemical processes for the preparation of pesticidal thioethers and pesticidal sulfoxides. Further, the present application relates to certain novel compounds necessary for their synthesis. It would be advantageous to produce pesticidal thioethers and pesticidal sulfoxides efficiently and in high yield from commercially available starting materials.

### BACKGROUND

There are more than ten thousand species of pests that cause losses in agriculture. The world-wide agricultural losses amount to billions of U.S. dollars each year. Stored food pests eat and adulterate stored food. The world-wide stored food losses amount to billions of U.S. dollars each year, but more importantly, deprive people of needed food. Certain pests have developed resistance to pesticides in current use. Hundreds of pest species are resistant to one or more pesticides. The development of resistance to some of the older pesticides, such as DDT, the carbamates, and the organophosphates, is well known. But resistance has even developed to some of the newer pesticides. As a result, there is an acute need for new pesticides that has led to the development of new pesticides. Specifically, US 20130288893(A1) describes, *inter alia,* certain pesticidal thioethers and their use as pesticides. Such compounds are finding use in agriculture for the control of pests.

In US 2015/0112077 A1, WO 2015/058028 A1 and United States Patent Number 9102654, processes for preparing such pesticidal thioethers were described. In one embodiment, the intermediate **Id** was prepared according to the process shown in Scheme 1 below.

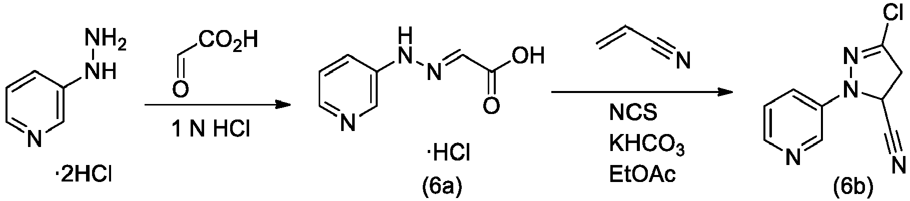

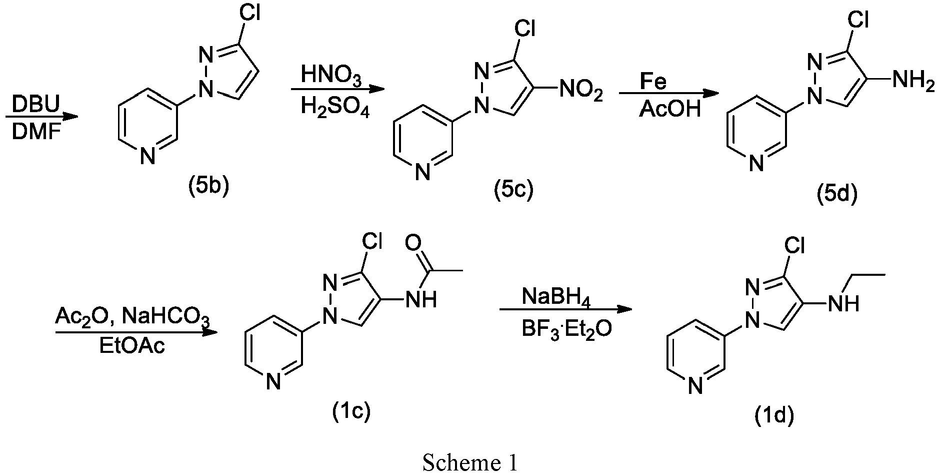

The process in Scheme 1 requires seven steps from commercially available starting material 3-hydrazinopyridine dihydrochloride to arrive at the compound of the formula **1d**, by way of compound **5d,** making the large scale manufacture of the target pesticidal thioethers described in United States Patent Number 9102654 difficult.

US 2012/0095023 A1 describes a process for preparing 1-pyridin-3-yl-1H-pyrazol-4-ylamine by coupling 4-nitro-1H-pyrazole with 3-bromopyridine and then reducing the nitro group. US 2013/0109566 A1 describes a process for preparing 1-pyridin-3-yl-1H-pyrazol-4-ylamine by first reacting pyrazole with 3-bromopyridine in the presence of cesium carbonate, copper oxide and salicylaldoxime, then nitrating the resulting 3-pyrazol-1-yl-pyridine, and finally reducing nitro group.

US 2015/0112074 A1 and US 2016/0152594 A1 describe the heteroarylation of N-(3-chloro-1H-pyrazol-4-ayl)acetamide using 3-bromopyridine.

Because there is a need for very large quantities of pesticides, particularly pesticidal thioethers of the type described in United States Patent Number 9102654 and US Patent Publication 20130288893(A1), it would be highly advantageous to develop new processes to produce pesticidal thioethers efficiently and in high yield from economical commercially available starting materials.

### DEFINITIONS OF THE DISCLOSURE

The following definitions apply to the terms as used throughout this specification, unless otherwise limited in specific instances.

As used herein, the term "alkyl" includes a chain of carbon atoms, which is optionally branched including but not limited to C₁-C₆, C₁-C₄, and C₁-C₃. Illustrative alkyl groups include, but are not limited to, methyl, ethyl, n-propyl, isopropyl, n-butyl, isobutyl, sec-butyl, tert-butyl, pentyl, 2-pentyl, and 3-pentyl. Alkyl may be substituted or unsubstituted. It will be understood that "alkyl" may be combined with other groups, such as those provided above, to form a functionalized alkyl. By way of example, the combination of an "alkyl" group, as described herein, with a "cycloalkyl" group may be referred to as a "alkyl-cycloalkyl" group.

As used herein, the term "cycloalkyl" refers to an all-carbon cyclic ring, optionally containing one or more double bonds but the cycloalkyl does not contain a completely conjugated pi-electron system. It will be understood that in certain embodiments, cycloalkyl may be advantageously of limited size, such as C₃-C₆. Cycloalkyl may be unsubstituted or substituted. Examples of cycloalkyl include cyclopropyl, cyclobutyl, and cyclohexyl.

As used herein, the term "aryl" refers to an all-carbon cyclic ring containing a completely conjugated pi-electron system. It will be understood that in certain embodiments, aryl may be advantageously of limited size, such as C₆-C₁₀. Aryl may be unsubstituted or substituted. Examples of aryl include phenyl and naphthyl.

As used herein, "halo" or "halogen" or "halide" may be used interchangeably and refers to fluorine (F), chlorine (Cl), bromine (Br) or iodine (I).

As used herein, "trihalomethyl" refers to a methyl group having three halo substituents, such as a trifluoromethyl group.

### DETAILED DESCRIPTION OF THE DISCLOSURE

The compounds and process of the present disclosure are described in detail below. The processes of the present disclosure can be described according to Scheme 2.

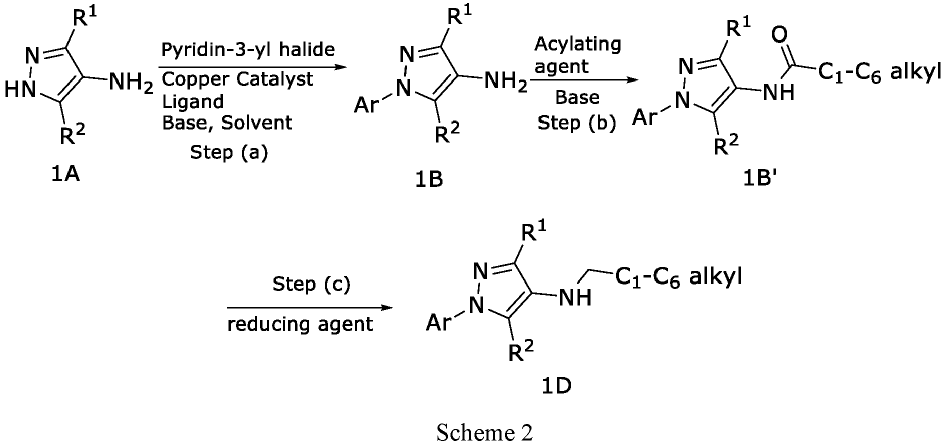

In Step (a) of Scheme 2, the pyrazole starting material 1A, wherein each of R¹ and R² is independently selected from the group consisting of H, F, Cl, Br, I, C₁-C₆ alkyl, and trifluoromethyl; can be reacted with a 3-halopyridine in the presence of a copper catalyst, a ligand, a base, a solvent and optionally an additive. The catalyst can be a copper (I) reagent or a copper (II) reagent. Exemplary catalysts include, but are not limited to, copper (I) chloride (CuCl), copper (II) chloride (CuCh), and copper (I) iodide (CuI). In some embodiments, the copper reagent is copper (I) chloride (CuCl). In some embodiments, the reaction can be carried out in the presence of 0.01 to 0.4 molar equivalents of copper catalyst compared to the pyrazole starting material. In some embodiments, the reaction can be carried out in the presence of 0.05 to 0.25 molar equivalents of copper catalyst compared to the pyrazole starting material. In some embodiments, the reaction can be carried out in the presence of 0.2 molar equivalents of copper catalyst compared to the pyrazole starting material.

The base in Step (a) can be an inorganic base. Exemplary suitable bases for use in connection with Step (a) include but are not limited sodium bicarbonate (NaHCO₃), sodium carbonate (Na₂CO₃), calcium carbonate (CaCO₃), cesium carbonate (Cs₂CO₃), lithium carbonate (Li₂CO₃), potassium carbonate (K₂CO₃), lithium hydroxide (LiOH), sodium hydroxide (NaOH), potassium hydroxide (KOH), cesium hydroxide (CsOH), calcium hydroxide (Ca(OH)₂), sodium diphosphate (Na₂HPO₄), sodium phosphate (Na₃PO₄), potassium diphosphate (Na₂HPO₄), potassium phosphate (K₃PO₄), sodium methoxide (NaOCH₃), and sodium ethoxide (NaOCH₂CH₃). In some embodiments, the base is K₃PO₄ or K₂CO₃. In some embodiments, it can be advantageous to use the base in excess compared to the pyrazole starting material. In some embodiments, the base is used in a 2-fold to a 5-fold excess. In some embodiments, the base is used in a 2-fold to a 3-fold excess. In some embodiments, the base is used in a 2-fold to excess.

The ligand in the process of Step (a) can be an amine or heteroaryl amine, such as *N,N'-*dimethylethane-1,2-diamine (DMEDA), triethylenetetreamine (TETA), *N*,*N*'-bis(2-hydroxyethyl)ethylenediamine (BHEEA) and 8-hydroxyquinoline. In some embodiments, the reaction can be carried out in the presence of less than an equimolar amount of the ligand to provide further reduction in costs. In some embodiments, the reaction can be carried out in the presence of 0.08 to 1.0 molar equivalents of ligand compared to the pyrazole starting material. In some embodiments, the reaction can be carried out in the presence of 0.4 to 0.6 molar equivalents of the ligand compared to the pyrazole starting material. In some embodiments, the reaction can be carried out in the presence of 0.1 to 0.2 molar equivalents of the ligand compared to the pyrazole starting material.

The process of Step (a) can be conducted in a solvent, such as, acetonitrile (CH₃CN), dioxane, *N*,*N*-dimethylformamide (DMF), *N*-methyl-2-pyrrolidone (NMP), tetrahydrofuran (THF), and toluene. In some embodiments, the solvent is dioxane. In some embodiments, it can be advantageous to carry out the reaction of Step (a) at an elevated temperature. In some embodiments, the reaction is carried out at a temperature between 50 °C and 150 °C. In some embodiments, the reaction is carried out at a temperature between 60 °C and 120 °C. In some embodiments, the reaction is carried out at a temperature between 95 °C and 115 °C.

The process of Step (b) can optionally be carried out when the product of Step (a) is, for example, compound 1B, by acylating the amine to provide a compound of the formula 1B'. In step (b), exemplary acylating agents include acetyl chloride or acetic anhydride. The base in the process of step (b) can be an inorganic base, such as sodium bicarbonate (NaHCO₃). Step (b) can be carried out in the presence of a solvent. In some embodiments, the solvent of step (b) is ethyl acetate (EtOAc) or tetrahydrofuran (THF). In some embodiments, it can be advantageous to carry out the process of Step (b) at a reduced temperature. In some embodiments, the reaction can be carried out at a temperature of -10 °C to 30 °C. In some embodiments, the base is used in a 5% molar excess to a 5-fold excess. In some embodiments, the base is used in a 2-fold to a 3-fold excess. In some embodiments, the base is used in a 2-fold to excess.

The process of Step (c) can be carried out by a reducing agent, such as borane, sodium borohydride (NaBH₄)/boron trifluoride diethyl etherate (BF₃•Et₂O), and sodium bis(2-methoxyethoxy)aluminumhydride (Red-Al). In some embodiments, it can be advantageous to carry out the reaction of Step (c) in the presence of from 2.0 to 5.0 molar equivalents of a reducing agent, such as sodium bis(2-methoxyethoxy)aluminumhydride (Red-Al). In some embodiments, the amount of Red-Al used in the process of Step (c) is 3.0 molar equivalents. The process of Step (c) can be carried out in the presence of a solvent or a mixture of solvents. In some embodiments, the solvent is tetrahydrofuran (THF), dioxane, diethyl ether (Et₂O), cyclopentylmethylether, or a mixture thereof. In some embodiments, the solvent is a mixture of THF and toluene. It can be advantageous to carry out the reaction of Step (c) at a temperature of from 0 °C to 80 °C. In some embodiments, it can be advantageous to carry out the process of Step (c) at an elevated temperature. In some embodiments, the temperature of Step (c) can be from 25 °C to 50 °C.

Alternatively, the processes of the present disclosure can be described according to Scheme 3.

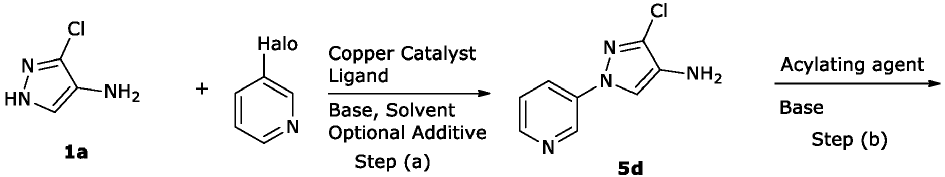

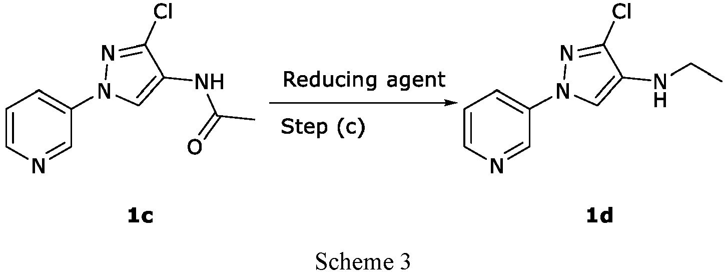

In Step (a) of Scheme 3, the pyrazole starting material la, can be reacted with 3-bromopyridine or 3-iodopyridine in the presence of a catalyst, a ligand, a base, and a solvent. The catalyst can be a copper (I) reagent or a copper (II) reagent. Exemplary catalysts include, but are not limited to, copper (I) chloride (CuCl), copper (II) chloride (CuCh), and copper (I) iodide (CuI). In some embodiments, the copper reagent is copper (I) chloride (CuCl). In some embodiments, the reaction can be carried out in the presence of 0.01 to 0.4 molar equivalents of copper catalyst compared to the pyrazole starting material. In some embodiments, the reaction can be carried out in the presence of 0.1 to 0.25 molar equivalents of copper catalyst compared to the pyrazole starting material. In some embodiments, the reaction can be carried out in the presence of 0.2 molar equivalents of copper catalyst compared to the pyrazole starting material.

The base in Step (a) can be an inorganic base. Exemplary suitable bases for use in connection with Step (a) include but are not limited sodium bicarbonate (NaHCO₃), sodium carbonate (Na₂CO₃), calcium carbonate (CaCO₃), cesium carbonate (Cs₂CO₃), lithium carbonate (Li₂CO₃), potassium carbonate (K₂CO₃), lithium hydroxide (LiOH), sodium hydroxide (NaOH), potassium hydroxide (KOH), cesium hydroxide (CsOH), calcium hydroxide (Ca(OH)₂), sodium diphosphate (Na₂HPO₄), sodium phosphate (Na₃PO₄), potassium diphosphate (K₂HPO₄), potassium phosphate (K₃PO₄), sodium methoxide (NaOCH₃), and sodium ethoxide (NaOCH₂CH₃). In some embodiments, the base is K₃PO₄ or K₂CO₃. In some embodiments, it can be advantageous to use the base in excess compared to the pyrazole starting material. In some embodiments, the base is used in a 2-fold to a 5-fold excess. In some embodiments, the base is used in a 2-fold to a 3-fold excess. In some embodiments, the base is used in a 2-fold to excess.

The ligand in the process of Step (a) can be an amine or heteroaryl amine, such as *N,N'-*dimethylethane-1,2-diamine (DMEDA), triethylenetetreamine (TETA), *N*,*N*'-bis(2-hydroxyethyl)ethylenediamine (BHEEA) and 8-hydroxyquinoline. In some embodiments, the reaction can be carried out in the presence of 0.08 to 1.0 molar equivalents of ligand compared to the pyrazole starting material. In some embodiments, the reaction can be carried out in the presence of 0.4 to 0.6 molar equivalents of the ligand compared to the pyrazole starting material. In some embodiments, the reaction can be carried out in the presence of 0.1 to 0.2 molar equivalents of the ligand compared to the pyrazole starting material.

The process of Step (a) can be conducted in a solvent, such as, acetonitrile (CH₃CN), dioxane, *N*,*N*-dimethylformamide (DMF), *N*-methyl-2-pyrrolidone (NMP), tetrahydrofuran (THF), and toluene. In some embodiments, the solvent is dioxane. In some embodiments, it can be advantageous to carry out the reaction of Step (a) at an elevated temperature. In some embodiments, the reaction is carried out at a temperature between 50 °C and 150 °C. In some embodiments, the reaction is carried out at a temperature between 60 °C and 120 °C. In some embodiments, the reaction is carried out at a temperature between 95 °C and 115 °C.

In step (b), exemplary acylating agents include acetyl chloride or acetic anhydride. The base in the process of step (b) can be an inorganic base, such as sodium bicarbonate (NaHCO₃), sodium carbonate (Na₂CO₃), calcium carbonate (CaCO₃), cesium carbonate (Cs₂CO₃), lithium carbonate (Li₂CO₃), potassium carbonate (K₂CO₃), lithium hydroxide (LiOH), sodium hydroxide (NaOH), potassium hydroxide (KOH), cesium hydroxide (CsOH), calcium hydroxide (Ca(OH)₂), sodium diphosphate (Na₂HPO₄), and potassium phosphate (K₃PO₄). In some embodiments, the base in step (b) can be sodium bicarbonate (NaHCO₃). Step (b) can be carried out in the presence of a solvent, such as methylene dichloride (DCM), *N,N-*dimethylformamide (DMF), tetrahydrofuran (THF), ethyl acetate (EtOAc), acetone, acetonitrile (CH₃CN), and dimethylsulfoxide (DMSO). In some embodiments, the solvent of step (b) is ethyl acetate (EtOAc) or tetrahydrofuran (THF). In some embodiments, it can be advantageous to carry out the process of Step (b) at a reduced temperature. In some embodiments, the reaction can be carried out at a temperature of -10 °C to 30 °C. In some embodiments, the base is used in a 5% molar excess to a 5-fold excess. In some embodiments, the base is used in a 2-fold to a 3-fold excess. In some embodiments, the base is used in a 2-fold to excess.

The process of Step (c) can be carried out by a reducing agent, such as borane, sodium borohydride (NaBH₄)/boron trifluoride diethyl etherate (BF₃•Et₂O), and sodium bis(2-methoxyethoxy)aluminumhydride (Red-Al). In some embodiments, it can be advantageous to carry out the reaction of Step (c) in the presence of from 2.0 to 5.0 molar equivalents of a reducing agent, such as sodium bis(2-methoxyethoxy)aluminumhydride (Red-Al). In some embodiments, the amount of sodium bis(2-methoxyethoxy)aluminumhydride (Red-Al) used in the process of Step (c) is 3.0 molar equivalents. The process of Step (c) can be carried out in the presence of a solvent or a mixture of solvents. In some embodiments, the solvent is tetrahydrofuran (THF), dioxane, diethyl ether (Et₂O), cyclopentylmethylether, or a mixture thereof. In some embodiments, the solvent is a mixture of THF and toluene. It can be advantageous to carry out the reaction of Step (c) at a temperature of from 0 °C to 80 °C. In some embodiments, it can be advantageous to carry out the process of Step (c) at an elevated temperature. In some embodiments, the temperature of Step (c) can be from 25 °C to 50 °C.

Exemplary methods for the preparation of pesticidal thioethers from compound **Id** can be found in, for example, United States Patent Number 9102654. Exemplary embodiments of such proceses can be described as shown in Scheme 4.

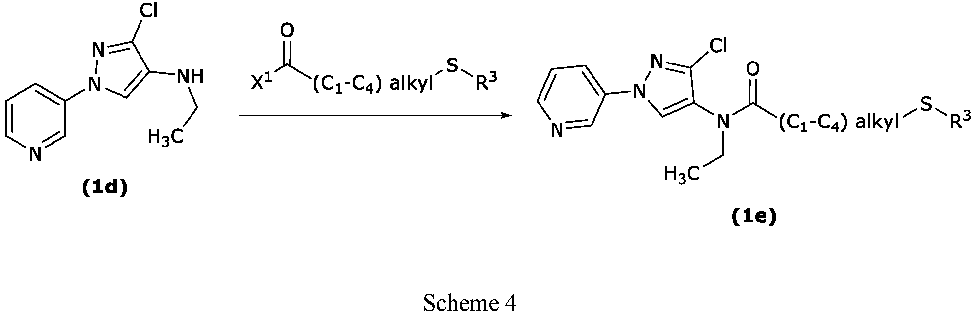

In Scheme 4, 3-chloro-*N*-ethyl-1-(pyridin-3-yl)-1*H*-pyrazol-amine (1d) is acylated with activated carbonyl thioethers, indicated as X¹C(=O)(C₁-C₄)-alkyl-S-R³, to produce pesticidal thioethers (1e). In some embodiments, R³ is (C₁-C₄)-haloalkyl, In some embodiments, R³ is CH₂CH₂CF₃.

When X¹ is Cl, the reaction is conducted in a solvent such as ethyl acetate. The reaction may be optionally conducted in the presence of a base such, as sodium bicarbonate, to yield pesticidal thioethers (1e).

When X¹ is OC(=O)(C₁-C₄)-alkyl, the reaction is conducted in the presence of a base preferably sodium bicarbonate, to yield pesticidal thioethers (1e). Alternatively, the reaction may be conducted when X¹ is an activated carboxylic acid, activated by such reagents as 2,4,6-tripropyl-trioxatriphosphinane-2,4,-trioxide (T₃P), carbonyldiimidazole (CDI), dicyclohexylcarbodiimide (DCC) or 1-ethyl-3-(3-dimethyl-aminopropyl)carbodiimide (EDC), preferably 2,4,6-tripropyl-trioxatriphosphinane-2,4,-trioxide and carbonyldiimidazole at temperatures of 0 °C to 80 °C; this reaction may also be conducted with uronium or phosphonium activating groups such as O-(7-azabenzotriazol-1-yl)-*N*,*N*,*N'*,*N'-*tetramethyluronium hexafluorophosphate (HATU) or benzotriazol-1-yl-oxytripyrrolidino-phosphonium hexafluorophosphate (PyBOP), in the presence of an amine base such as diisopropylethylamine or triethylamine, in an aprotic solvent such as *N,N*-dimethylformamide, tetrahydrofuran, or dichloromethane, at temperatures of -10 °C to 30 °C, to form pesticidal thioethers (1e).

Activated carbonyl thioethers are prepared from X¹C(=O)(C₁-C₄)-alkyl-S-R³ wherein X¹ is OH, which are prepared by saponifying the corresponding ester thioethers, indicated as X¹C(=O)(C₁-C₄)-alkyl-S-R³, wherein X¹ is O(C₁-C₄)-alkyl, with a metal hydroxide such as lithium hydroxide, in a solvent such as methanol or tetrahydrofuran. Alternatively, X¹C(=O)(C₁-C₄)-alkyl-S-R³, wherein X¹ is OH or O(C₁-C₄)-alkyl may be prepared by the photochemical free-radical coupling of 3-mercaptopropionic acid and esters thereof with 3,3,3-trifluoropropene in the presence of 2,2-dimethoxy-2-phenylacetophenone initiator and long wavelength UV light in an organic solvent. Additionally, X¹C(=O)(C₁-C₄)-alkyl-S-R³, wherein X¹ is OH or O(C₁-C₄)-alkyl may also be prepared by the low temperature free-radical initiated coupling of 3-mercaptopropionic acid and esters thereof with 3,3,3-trifluoropropene in the presence of 2,2'-azobis(4-methoxy-2,4-dimethyl) valeronitrile (V-70) initiator at temperatures of -50 °C to 40 °C in a solvent. Preferably, X¹C(=O)(C₁-C₄)-alkyl-S-R³, wherein X¹ is OH or O(C₁-C₄)-alkyl, is prepared by the low temperature free-radical initiated coupling of 3-mercaptopropionic acid and esters thereof with 3,3,3-trifluoropropene in the presence of a two component initiator system of benzoyl peroxide and dimethylaniline or N-phenyldiethanolamine at temperatures of -50 °C to 40 °C in a solvent such as toluene or ethyl acetate.

In some embodiments, the processes described here in comprise Step (a) and Step (b). In some embodiments, the processes described here in comprise Step (a) and Step (c). In some embodiments, the processes described here in comprise Step (a), Step (b) and Step (c). In some embodiments, the processes of the present disclosure can be carried out in connection with processes for preparing pesticidal thioethers, such as those described in United States Patent Number 9102654.

### EXAMPLES

Starting materials, reagents, and solvents that were obtained from commercial sources were used without further purification. Melting points are uncorrected. Examples using "room temperature" were conducted in climate controlled laboratories with temperatures ranging from 20 °C to 24 °C. Molecules are given their known names, named according to naming programs within Accelrys Draw, ChemDraw, or ACD Name Pro. If such programs are unable to name a molecule, such molecule is named using conventional naming rules. ¹H NMR spectral data are in ppm (δ) and were recorded at 300, 400, 500, or 600 MHz; ¹³C NMR spectral data are in ppm (δ) and were recorded at 75, 100, or 150 MHz, and ¹⁹F NMR spectral data are in ppm (δ) and were recorded at 376 MHz, unless otherwise stated.

3-Chloro-1*H*-pyrazol-4-amine hydrochloride, compound la, was prepared according to the method described in United States Patent Number 9,102,655.

### COMPOUND EXAMPLES

### Example 1 - Preparation of 3-chloro-1(pyridin-3-yl)-1H-pyrazol-4-amine (5d)

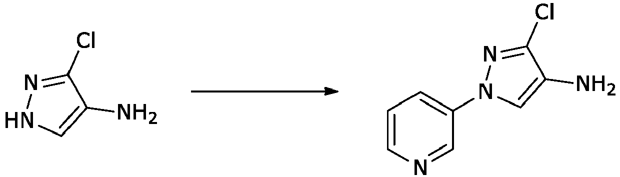

A three-neck round bottomed flask (100 mL) was charged with copper (I) chloride (0.627 g, 6.33 mmol), *N,N*'-dimethylethane-1,2-diamine (1.12 g, 12.7 mmol), 3-chloro-1*H-*pyrazol-4-amine hydrochloride (5.85 g, 38.0 mmol), potassium carbonate (8.75 g, 63.3 mmol), and dioxane (50 mL), and the mixture was stirred under nitrogen for 10 min. 3-Bromopyridine (3.05 mL, 31.6 mmol) was added, and the mixture was heated at 80 °C for 18 h. The reaction was allowed to cool to 20 °C and filtered through a Celite^{®} pad. The pad was rinsed with ethyl acetate (2 × 20 mL) and the combined filtrates were concentrated. Purification by flash column chromatography using 0-80% ethyl acetate/hexanes as eluent provided the title compound as a light yellow solid (3.56 g, 58%): ¹H NMR (400 MHz, DMSO-*d*₆) δ 8.95 (dd, *J* = 2.6, 0.8 Hz, 1H), 8.45 (dd, *J=* 4.7, 1.4 Hz, 1H), 1H), 8.07 (ddd, *J=* 8.4, 2.4, 1.4 Hz, 1H), 7.90 (s, 1H), 7.48 (ddd, *J=* 8.3, 4.7, 0.8 Hz, 1H), 4.42 (s, 2H); ¹³C NMR (101 MHz, DMSO-*d*₆) δ 146.35, 138.53, 135.72, 132.09, 130.09, 124.29, 124.11, 114.09; ESIMS *m*/*z* 195 ([M+H])⁺).

### Example 2 - Preparation of N-(3-Chloro-1-(pyridin-3-yl)-1H-pyrazol-4-yl)acetamide (1c)

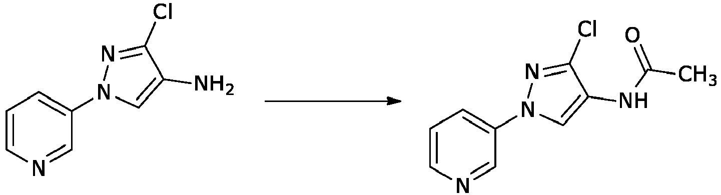

A three-neck round bottomed flask (100 mL) was charged with 3-chloro-1(pyridin-3-yl)-1*H*-pyrazol-4-amine (1.00 g, 5.14 mmol) and ethyl acetate (10 mL). Sodium bicarbonate (1.08 g, 12.9 mmol) was added, followed by dropwise addition of acetic anhydride (0.629 g, 6.17 mmol) at <20 °C. The reaction was stirred at 20 °C for 2 h to afford a suspension, at which point thin layer chromatography analysis [TLC, eluent: ethyl acetate] indicated that the reaction was complete. The reaction was diluted with water (50 mL) and the resulting suspension was filtered. The solid was rinsed with water (10 mL) followed by methanol (5 mL). The solid was further dried under vacuum at 20 °C to afford the desired product as a white solid (0.804 g, 66%): mp 169-172 °C; ¹H NMR (400 MHz, DMSO-*d*₆) δ 9.84 (s, 1H), 9.05 (dd, *J=* 2.8, 0.8 Hz, 1H), 8.82 (s, 1H), 8.54 (dd, *J* = 4.7, 1.4 Hz, 1H), 8.20 (ddd, *J* = 8.4, 2.8, 1.4 Hz, 1H), 7.54, (ddd, *J=* 8.3, 4.7, 0.8 Hz, 1H), 2.11 (s, 3H); ¹³C NMR (101 MHz, DMSO-*d*₆) δ 168,12, 147,46, 139,42, 135.46, 133.60, 125.47, 124.21, 122.21, 120,16, 22.62; EIMS *m*/*z* 236 ([M]⁺).

### Example 3 - Preparation of 3-chloro-N-ethyl-1-(pyridin-3-yl)-1H-pyrazol-amine

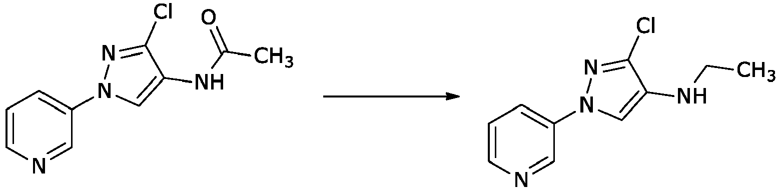

To a 25 mL round bottom flask was added *N*-(3-chloro-1-(pyridin-3-yl)-1*H*-pyrazol-4-yl)-*N*-ethylacetamide (1.0 g, 4.2 mmol), and anhydrous THF (6.0 mL) leading to a white suspension. The suspension was cooled in an ice-water bath to 6 °C. Sodium bis(2-methoxyethoxy)aluminum dihydride (Red-Al, 60 wt% in toluene, 3.52 mL, 2.5 eq.) was added slowly with via syringe over 10 min while keeping pot temp <10 °C. The thick suspension gradually turned clear yellow solution during Red-Al addition. The reaction mixture was slowly warmed up to 25 °C over 1.5 h. LC indicated 83.8% conversion. The solution was heated to 50 °C and stirred for 4.5 h. LC indicated 88.1% conversion. The solution was cooled down to 20 °C. NaOH (2 M in H₂O, 5 mL) was added to quench the reaction leading to white suspension. Water (20 mL) was added and the mixture separated into two phases. The aqueous phase was separated and extracted with EtOAc (2 × 20 mL). The organic layers were combined, dried over anhydrous sodium sulfate, and concentrated by rotavap to afford a crude product as a red-brown oil (0.758 g). LC assay using di-*n*-propyl phthalate as internal standard indicated 78 wt%, 0.591 g active, 62.8% yield. ESIMS m/z 222 ([M+H]⁺).

### Example 4 - Alternative preparation of 3-chloro-N-ethyl-1-(pyridin-3-yl)-1H-pyrazol-amine

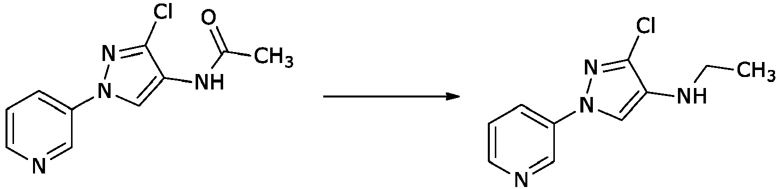

To a 25 mL round bottom flask was added *N*-(3-chloro-1-(pyridin-3-yl)-1*H*-pyrazol-4-yl)-*N*-ethylacetamide (1.0 g, 4.2 mmol) and anhydrous THF (9 mL) leading to a while suspension. The flask was cooled in an ice-water bath to 5 °C. Red-Al (60 wt% in toluene, 4.3 mL, 3.0 eq.) was added slowly via syringe over 15 min while keeping pot temp <10 °C. The thick suspension gradually turned clear yellow solution during the addition of the Red-Al. The reaction mixture was slowly warmed up to 25 °C over 3 h, then was stirred at 25 °C for 17 h. NaOH (2 M in H₂O, 6 mL) was added to quench the reaction leading to a thick slurry. Water (20 mL) was added and the two layers were separated. The bottom aqueous phase was extracted with Et₂O (3 × 20 mL). Combined organic layers were dried over anhydrous sodium sulfate and concentrated by rotavap to afford crude product as red-brown oil 0.91 g. LC assay using di-n-propyl phthalate as the internal standard indicated 61 wt%, 0.555 g active, 59.0% yield. ESIMS m/z 222 ([M+H]⁺).

Alternative preparation of 3-Chloro-*N*-ethyl-1-(pyridin-3-yl)-1*H*-pyrazol-4-amine

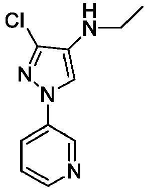

A 100 mL 3-neck round bottom flask was charged with *N*-(3-chloro-1-(pyridin-3-yl)-1*H*-pyrazol-4-yl)acetamide (475 mg, 2.01 mmol) and tetrahydrofuran (10 mL). Borontrifluoride etherate (0.63 mL, 5.02 mmol) was added and the mixture was stirred for 15 min to give a suspension. Sodium borohydride (228 mg, 6.02 mmol) was added and the reaction was heated at 60 °C for 4 h, at which point thin layer chromatography analysis [Eluent: ethyl acetate, sample was prepared by treatment of reaction mixture with hydrochloric acid, followed by sodium bicarbonate basification and ethyl acetate extraction] indicated that the reaction was complete. Water (10 mL) and concentrated hydrochloric acid (1 mL) were added and the reaction was heated at 60 °C for 1 h. The reaction mixture was cooled to room temperature and distilled to remove tetrahydrofuran. The mixture was neutralized with saturated aqueous sodium bicarbonate to pH ~8 to afford a suspension, which was stirred for 1 h and filtered. The filter cake was rinsed with water (10 mL) and dried under vacuum to afford a white solid (352 mg, 79%): mp 93-96 °C; ¹H NMR (400 MHz, DMSO-*d*₆) δ 8.99 (d, *J=* 2.7 Hz, 1H), 8.44 (dd, *J* = 4.6, 1.4 Hz, 1H), 8.10 (ddd, *J* = 8.4, 2.7, 1.4 Hz, 1H), 8.06 (s, 1H), 7.50 (dd, *J=* 8.4, 4.7 Hz, 1H), 4.63 (t, *J* = 6.0 Hz, 1H), 3.06-2.92 (m, 2H), 1.18 (t, *J* = 7.1 Hz, 3H); ¹³C NMR (101 MHz, DMSO-*d*₆) δ 146.17, 138.31, 135.81, 132.82, 130.84, 124.10, 123.96, 112.23, 40.51, 14.28; EIMS *m*/*z* 222 ([M]⁺).

### Example 5 - Preparation of N-(3-Chloro-1-(pyridin-3-yl)-1H-pyrazol-4-yl)-N-ethyl-3-((3,3,3-trifluoropropyl)thio)propanamide (Compound 6.9)

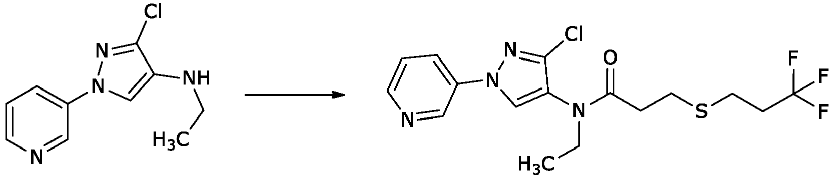

A three-neck round bottomed flask (100 mL) was charged with 3-chloro-*N*-ethyl-1-(pyridin-3-yl)-1*H*-pyrazol-amine (5.00 g, 22.5 mmol) and ethyl acetate (50 mL). Sodium bicarbonate (4.72 g, 56.1mmol) was added, followed by dropwise addition of 3-((3,3,3-trifluoropropyl)thio)propanoyl chloride (5.95 g, 26.9 mmol) at <20 °C for 2 h, at which point HPLC analysis indicated that the reaction was complete. The reaction was diluted with water (50 mL) (off-gassing) and the layers separated. The aqueous layer was extracted with ethyl acetate (20 mL) and the combined organic layers were concentrated to dryness to afford a light brown solid (10.1 g, quantitative). A small sample of crude product was purified by flash column chromatography using ethyl acetate as eluent to obtain an analytical reference sample: mp 79-81 °C; ¹H NMR (400 MHz, DMSO-*d₆*) δ 9.11 (d, *J=* 2.7 Hz, 1H), 8.97 (s, 1H), 8.60 (dd, *J* = 4.8, 1.4 Hz, 1H), 8.24 (ddd, *J* = 8.4, 2.8, 1.4 Hz, 1H), 7.60 (ddd, *J* = 8.4, 4.7, 0.8 Hz, 1H), 3.62 (q, *J=* 7.2 Hz, 2H), 2.75 (t, *J=* 7.0 Hz, 2H), 2.66 - 2.57 (m, 2H), 2.57 - 2.44 (m, 2H), 2.41 (t, *J=* 7.0 Hz, 2H), 1.08 (t, *J=* 7.1 Hz, 3H); ESIMS *m*/*z* 407 ([M+H]⁺).

## Claims

1. A process comprising
(a) contacting a compound of the formula 1A 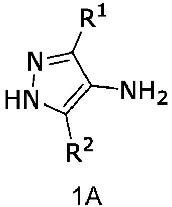 wherein each of R¹ and R² is independently selected from the group consisting of H, F, Cl, Br, I, C₁-C₆ alkyl, and trifluoromethyl; with a 3-halopyridine in the presence of a catalyst, a ligand, a base and a solvent to provide a compound of the formula 1B 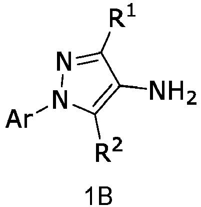 wherein Ar is pyridin-3-yl; and each of R¹ and R² is independently selected from the group consisting of H, F, Cl, Br, I, C₁-C₆ alkyl and trifluoromethyl.

2. The process of claim 1, further comprising
(b) contacting a compound of the formula 1B 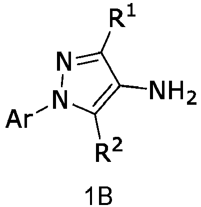 wherein Ar is pyridin-3-yl; and each of R¹ and R² is independently selected from the group consisting of H, F, Cl, Br, I, C₁-C₆ alkyl and trifluoromethyl;
with an acylating agent in the presence of a base and a solvent to provide a compound of the formula 1B' 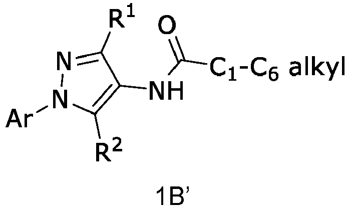 wherein Ar is pyridin-3-yl; and each of R¹ and R² is independently selected from the group consisting of H, F, Cl, Br, I, C₁-C₆ alkyl and trifluoromethyl.

3. The process of claim 1, wherein the catalyst in step (a) is copper (I) chloride (CuCI), copper (II) chloride (CuCl₂), copper (I) bromide (CuBr), or copper (I) iodide (CuI).

4. The process of claim 1, wherein the ligand in step (a) is selected from the group consisting of *N,N'*-dimethylethane-1,2-diamine (DMEDA), triethylenetetreamine (TETA), *N,N'*-bis(2-hydroxyethyl)ethylenediamine (BHEEA) and 8-hydroxyquinoline.

5. The process of claim 1, wherein the base in step (a) is selected from the group consisting of sodium bicarbonate (NaHCO₃), sodium carbonate (Na₂CO₃), calcium carbonate (CaCO₃), cesium carbonate (Cs₂CO₃), lithium carbonate (Li₂CO₃), potassium carbonate (K₂CO₃), lithium hydroxide (LiOH), sodium hydroxide (NaOH), potassium hydroxide (KOH), cesium hydroxide (CsOH), calcium hydroxide (Ca(OH)₂), sodium diphosphate (Na₂HPO₄), sodium phosphate (Na₃PO₄), potassium diphosphate (K₂HPO₄), potassium phosphate (K₃PO₄), sodium methoxide (NaOCH₃) and sodium ethoxide (NaOCH₂CH₃).

6. The process of claim 5, wherein the base in step (a) is K₂CO₃.

7. The process of claim 1, wherein the solvent in step (a) is acetonitrile (CH₃CN), dioxane, *N,N*-dimethylformamide (DMF), *N*-methyl-2-pyrrolidone (NMP), tetrahydrofuran (THF), toluene, or dimethylsulfoxide (DMSO).

8. The process of claim 2, wherein the acylating agent in step (b) is acetyl chloride or acetic anhydride.

9. The process of claim 2, wherein the base in step (b) is selected from the group consisting of sodium bicarbonate (NaHCO₃), sodium carbonate (Na₂CO₃), calcium carbonate (CaCO₃), cesium carbonate (Cs₂CO₃), lithium carbonate (Li₂CO₃), potassium carbonate (K₂CO₃), lithium hydroxide (LiOH), sodium hydroxide (NaOH), potassium hydroxide (KOH), cesium hydroxide (CsOH), calcium hydroxide (Ca(OH)₂), sodium diphosphate (Na₂HPO₄) and potassium phosphate (K₃PO₄).

10. The process of claim 9, wherein the base in step (b) is sodium bicarbonate (NaHCO₃).

11. The process of claim 2, wherein the solvent of step (b) is selected from the group consisting of methylene dichloride (DCM), *N,N*-dimethylformamide (DMF), tetrahydrofuran (THF), ethyl acetate (EtOAc), acetone, acetonitrile (CH₃CN) and dimethylsulfoxide (DMSO).

12. The process of claim 11, wherein the solvent is ethyl acetate (EtOAc) or tetrahydrofuran (THF).

13. The process of claim 1, wherein the 3-halopyridine is 3-bromopyridine.

14. The process of claim 1 or 2, wherein R² is H.

15. The process of claim 1 or 2, wherein R¹ is Cl.

## Patentansprüche

1. Ein Verfahren umfassend:
(a) In-Kontakt-Bringen einer Verbindung der Formel 1A 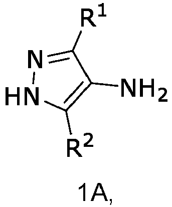 wobei R¹ und R² jeweils unabhängig voneinander aus der Gruppe bestehend aus H, F, Cl, Br, I, C₁-C₆-Alkyl und Trifluormethyl ausgewählt sind, mit einem 3-Halogenpyridin in Gegenwart eines Katalysators, eines Liganden, einer Base und eines Lösungsmittels, um eine Verbindung der Formel 1B bereitzustellen, 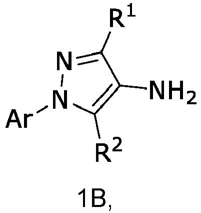 wobei Ar Pyridin-3-yl ist und R¹ und R² jeweils unabhängig voneinander aus der Gruppe bestehend aus H, F, Cl, Br, I, C₁-C₆-Alkyl und Trifluormethyl ausgewählt sind.

2. Das Verfahren gemäß Anspruch 1, des Weiteren umfassend:
(b) In-Kontakt-Bringen einer Verbindung der Formel 1B 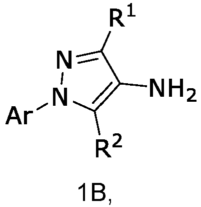 wobei Ar Pyridin-3-yl ist und R¹ und R² jeweils unabhängig voneinander aus der Gruppe bestehend aus H, F, Cl, Br, I, C₁-C₆-Alkyl und Trifluormethyl ausgewählt sind, mit einem Acylierungsmittel in Gegenwart einer Base und eines Lösungsmittels, um eine Verbindung der Formel 1B' bereitzustellen 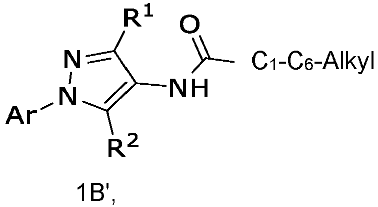 wobei Ar Pyridin-3-yl ist und R¹ und R² jeweils unabhängig voneinander aus der Gruppe bestehend aus H, F, Cl, Br, I, C₁-C₆-Alkyl und Trifluormethyl ausgewählt sind.

3. Das Verfahren gemäß Anspruch 1, wobei der Katalysator in Schritt (a) Kupfer(I)chlorid (CuCI), Kupfer(II)chlorid (CuCl₂), Kupfer(I)bromid (CuBr) oder Kupfer(I)iodid (Cul) ist.

4. Das Verfahren gemäß Anspruch 1, wobei der Ligand in Schritt (a) aus der Gruppe bestehend aus *N,N'*-Dimethylethylendiamin (DMEDA), Triethylentetramin (TETA), *N,N'*-Bis(2-hydroxyethyl)ethylendiamin (BHEEA) und 8-Hydroxychinolin ausgewählt ist.

5. Das Verfahren gemäß Anspruch 1, wobei die Base in Schritt (a) aus der Gruppe bestehend aus Natriumhydrogencarbonat (NaHCO₃), Natriumcarbonat (Na₂CO₃), Calciumcarbonat (CaCO₃), Caesiumcarbonat (Cs₂CO₃), Lithiumcarbonat (Li₂CO₃), Kaliumcarbonat (K₂CO₃), Lithiumhydroxid (LiOH), Natriumhydroxid (NaOH), Kaliumhydroxid (KOH), Caesiumhydroxid (CsOH), Calciumhydroxid (Ca(OH)₂), Natriumhydrogenphosphat (Na₂HPO₄), Natriumphosphat (Na₃PO₄), Kaliumhydrogenphosphat (K₂HPO₄), Kaliumphosphat (K₃PO₄), Natriummethanolat (NaOCH₃) und Natriumethanolat (NaOCH₂CH₃) ausgewählt ist.

6. Das Verfahren gemäß Anspruch 5, wobei die Base in Schritt (a) K₂CO₃ ist.

7. Das Verfahren gemäß Anspruch 1, wobei das Lösungsmittel in Schritt (a) Acetonitril (CH₃CN), Dioxan, *N,N*-Dimethylformamid (DMF), *N*-Methyl-2-pyrrolidon (NMP), Tetrahydrofuran (THF), Toluol oder Dimethylsulfoxid (DMSO) ist.

8. Das Verfahren gemäß Anspruch 2, wobei das Acylierungsmittel in Schritt (b) Acetylchlorid oder Essigsäureanhydrid ist.

9. Das Verfahren gemäß Anspruch 2, wobei die Base in Schritt (b) aus der Gruppe bestehend aus Natriumhydrogencarbonat (NaHCO₃), Natriumcarbonat (Na₂CO₃), Calciumcarbonat (CaCO₃), Caesiumcarbonat (Cs₂CO₃), Lithiumcarbonat (Li₂CO₃), Kaliumcarbonat (K₂CO₃), Lithiumhydroxid (LiOH), Natriumhydroxid (NaOH), Kaliumhydroxid (KOH), Caesiumhydroxid (CsOH), Calciumhydroxid (Ca(OH)₂), Natriumhydrogenphosphat (Na₂HPO₄) und Kaliumphosphat (K₃PO₄) ausgewählt ist.

10. Das Verfahren gemäß Anspruch 9, wobei die Base in Schritt (b) Natriumhydrogencarbonat (NaHCO₃) ist.

11. Das Verfahren gemäß Anspruch 2, wobei das Lösungsmittel in Schritt (b) aus der Gruppe bestehend aus Methylenchlorid (DCM), *N,N-*Dimethylformamid (DMF), Tetrahydrofuran (THF), Essigsäureethylester (EtOAc), Aceton, Acetonitril (CH₃CN) und Dimethylsulfoxid (DMSO) ausgewählt ist.

12. Das Verfahren gemäß Anspruch 11, wobei das Lösungsmittel Essigsäureethylester (EtOAc) oder Tetrahydrofuran (THF) ist.

13. Das Verfahren gemäß Anspruch 1, wobei das 3-Halogenpyridin 3-Brompyridin ist.

14. Das Verfahren gemäß Anspruch 1 oder 2, wobei R² H ist.

15. Das Verfahren gemäß Anspruch 1 oder 2, wobei R¹ Cl ist.

## Revendications

1. Procédé comportant
a) le fait de mettre en contact un composé de formule 1A 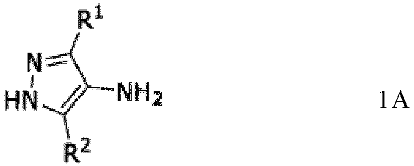 dans laquelle chacune des entités représentées par R¹ et R² est choisie indépendamment dans l'ensemble formé par les atomes d'hydrogène, de fluor, de chlore, de brome et d'iode et les groupes alkyle en C₁-C₆ et trifluorométhyle,
et une 3-halogéno-pyridine, en présence d'un catalyseur, d'un ligand, d'une base et d'un solvant, pour obtenir un composé de formule 1B 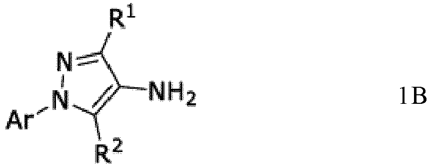 dans laquelle Ar représente un groupe pyridin-3-yle, et chacune des entités représentées par R¹ et R² est choisie indépendamment dans l'ensemble formé par les atomes d'hydrogène, de fluor, de chlore, de brome et d'iode et les groupes alkyle en C₁-C₆ et trifluorométhyle.

2. Procédé conforme à la revendication 1, qui comporte en outre a) le fait de mettre en contact un composé de formule 1B 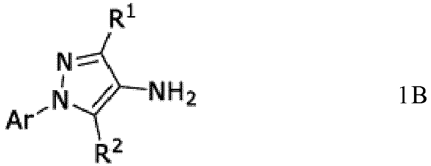 dans laquelle Ar représente un groupe pyridin-3-yle, et chacune des entités représentées par R¹ et R² est choisie indépendamment dans l'ensemble formé par les atomes d'hydrogène, de fluor, de chlore, de brome et d'iode et les groupes alkyle en C₁-C₆ et trifluorométhyle,
et un agent d'acylation, en présence d'une base et d'un solvant, pour obtenir un composé de formule 1B' 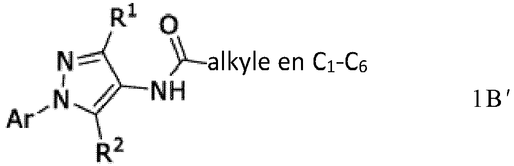 dans laquelle Ar représente un groupe pyridin-3-yle, et chacune des entités représentées par R¹ et R² est choisie indépendamment dans l'ensemble formé par les atomes d'hydrogène, de fluor, de chlore, de brome et d'iode et les groupes alkyle en C₁-C₆ et trifluorométhyle.

3. Procédé conforme à la revendication 1, dans lequel le catalyseur utilisé dans l'étape (a) est du chlorure de cuivre-I (CuCl), du chlorure de cuivre-II (CuCl₂), du bromure de cuivre-I (CuBr) ou du iodure de cuivre-I (CuI).

4. Procédé conforme à la revendication 1, dans lequel le ligand utilisé dans l'étape (a) est choisi dans l'ensemble formé par la N,N'-diméthyl-éthane-1,2-diamine (DMEDA), la triéthylène-tétramine (TETA), la N,N'-bis(2-hydroxy-éthyl)-éthylène-diamine (BHEEA) et la 8-hydroxy-quinoléine.

5. Procédé conforme à la revendication 1, dans lequel la base utilisée dans l'étape (a) est choisie dans l'ensemble formé par le bicarbonate de sodium (NaHCO₃), le carbonate de sodium (Na₂CO₃), le carbonate de calcium (CaCO₃), le carbonate de césium (Cs₂CO₃), le carbonate de lithium (Li₂CO₃), le carbonate de potassium (K₂CO₃), l'hydroxyde de lithium (LiOH), l'hydroxyde de sodium (NaOH), l'hydroxyde de potassium (KOH), l'hydroxyde de césium (CsOH), l'hydroxyde de calcium (Ca(OH)₂), l'hydrogénophosphate de sodium (Na₂HPO₄), le phosphate de sodium (Na₃PO₄), l'hydrogénophosphate de potassium (K₂HPO₄), le phosphate de potassium (K₃PO₄), le méthylate de sodium (NaOCH₃) et l'éthylate de sodium (NaOCH₂CH₃).

6. Procédé conforme à la revendication 5, dans lequel la base utilisée dans l'étape (a) est du K₂CO₃.

7. Procédé conforme à la revendication 1, dans lequel le solvant utilisé dans l'étape (a) est de l'acétonitrile (CH3CN), du dioxane, du N,N-diméthyl-formamide (DMF), de la N-méthyl-2-pyrrolidone (NMP), du tétrahydrofurane (THF), du toluène ou du diméthyl-sulfoxyde (DMSO).

8. Procédé conforme à la revendication 2, dans lequel l'agent d'acylation utilisé dans l'étape (b) est du chlorure d'acétyle ou de l'anhydride acétique.

9. Procédé conforme à la revendication 2, dans lequel la base utilisée dans l'étape (b) est choisie dans l'ensemble formé par le bicarbonate de sodium (NaHCO₃), le carbonate de sodium (Na₂CO₃), le carbonate de calcium (CaCO₃), le carbonate de césium (Cs₂CO₃), le carbonate de lithium (Li₂CO₃), le carbonate de potassium (K₂CO₃), l'hydroxyde de lithium (LiOH), l'hydroxyde de sodium (NaOH), l'hydroxyde de potassium (KOH), l'hydroxyde de césium (CsOH), l'hydroxyde de calcium (Ca(OH)₂), l'hydrogénophosphate de sodium (Na₂HPO₄) et le phosphate de potassium (K₃PO₄).

10. Procédé conforme à la revendication 9, dans lequel la base utilisée dans l'étape (b) est du bicarbonate de sodium (NaHCO₃).

11. Procédé conforme à la revendication 2, dans lequel le solvant utilisé dans l'étape (b) est choisi dans l'ensemble formé par du dichlorure de méthylène (DCM), du N,N-diméthyl-formamide (DMF), du tétrahydrofurane (THF), de l'acétate d'éthyle (EtOAc), de l'acétone, de l'acétonitrile (CH3CN) et du diméthyl-sulfoxyde (DMSO).

12. Procédé conforme à la revendication 11, dans lequel le solvant est de l'acétate d'éthyle (EtOAc) ou du tétrahydrofurane (THF).

13. Procédé conforme à la revendication 1, dans lequel la 3-halogéno-pyridine est de la 3-bromo-pyridine

14. Procédé conforme à la revendication 1 ou 2, dans lequel R² représente un atome d'hydrogène.

15. Procédé conforme à la revendication 1 ou 2, dans lequel R¹ représente un atome de chlore.
